(19) **Europäisches Patentamt European Patent Office Office européen des brevets**

(11) **EP 3 048 948 B1**

(12) **FASCICULE DE BREVET EUROPEEN**

(45) Date de publication et mention de la délivrance du brevet:
**20.06.2018 Bulletin 2018/25**

(21) Numéro de dépôt: **14777043.2**

(22) Date de dépôt: **24.09.2014**

(51) Int Cl.:
*A61B 3/113* (2006.01)    *A61B 5/00* (2006.01)
*A61B 5/0476* (2006.01)    *A61B 5/11* (2006.01)
*A61B 5/16* (2006.01)    *A61B 5/18* (2006.01)

(86) Numéro de dépôt international:
**PCT/EP2014/070392**

(87) Numéro de publication internationale:
**WO 2015/044216 (02.04.2015 Gazette 2015/13)**

(54) **PROCÉDÉ, SYSTÈME ET PROGRAMME D'ORDINATEUR POUR LA DÉTECTION ET LA CARACTÉRISATION DE CLIGNEMENTS OCULAIRES PAR ÉLECTRO-ENCÉPHALOGRAPHIE**

VERFAHREN, SYSTEM UND COMPUTERPROGRAMM ZUR ERKENNUNG UND CHARAKTERISIERUNG VON AUGENBLINZELN MITTELS ELEKTROENZEPHALOGRAFIE

METHOD, SYSTEM AND COMPUTER PROGRAM FOR DETECTION AND CHARACTERISATION OF OCULAR BLINKING BY ELECTROENCEPHALOGRAPHY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.09.2013 FR 1359288**

(43) Date de publication de la demande:
**03.08.2016 Bulletin 2016/31**

(73) Titulaire: **Commissariat à l'Énergie Atomique et aux Énergies Alternatives 75015 Paris (FR)**

(72) Inventeurs:
• **ROY, Raphaelle 31400 Touluse (FR)**
• **BONNET, Stéphane 69003 Lyon (FR)**
• **CHARBONNIER, Sylvie 38130 Echirolles (FR)**

(74) Mandataire: **Brevalex 95, rue d'Amsterdam 75378 Paris Cedex 8 (FR)**

(56) Documents cités:
**JP-A- 2001 061 801    US-A1- 2012 184 869**

• **ZHIYU QIAN ET AL: "Analysis of fatigue with 3D TV based on EEG", ORANGE TECHNOLOGIES (ICOT), 2013 INTERNATIONAL CONFERENCE ON, IEEE, 12 mars 2013 (2013-03-12), pages 306-309, XP032415434, DOI: 10.1109/ICOT.2013.6521219 ISBN: 978-1-4673-5934-4**
• **HSIAO-LUNG CHAN ET AL: "The Removal of Ocular Artifacts from EEG Signals Using Adaptive Filters Based on Ocular Source Components", ANNALS OF BIOMEDICAL ENGINEERING, KLUWER ACADEMIC PUBLISHERS-PLENUM PUBLISHERS, NE, vol. 38, no. 11, 8 juin 2010 (2010-06-08), pages 3489-3499, XP019811684, ISSN: 1573-9686**
• **CHAMBERS J ET AL: "Artifact Removal From Electroencephalograms Using a Hybrid BSS-SVM Algorithm", IEEE SIGNAL PROCESSING LETTERS, IEEE SERVICE CENTER, PISCATAWAY, NJ, US, vol. 12, no. 10, 1 octobre 2005 (2005-10-01), pages 721-724, XP011139117, ISSN: 1070-9908, DOI: 10.1109/LSP.2005.855539 cité dans la demande**

**Description**

**DOMAINE TECHNIQUE**

Le domaine de l'invention est celui de l'analyse de signaux électrophysiologiques d'une personne enregistrés par électro-encéphalographie (EEG). L'invention concerne plus particulièrement une analyse dédiée à l'identification d'artefacts dans les signaux EEG d'une personne pour détecter et caractériser les clignements oculaires de la personne.

**ÉTAT DE LA TECHNIQUE ANTÉRIEURE**

Une interface cerveau-machine (ICM, ou BCI pour « Brain-Computer Interface » en anglais) est un système permettant un échange d'informations entre le cerveau d'un individu et une machine extérieure à celui-ci, sans passer par une activité motrice mais en utilisant comme entrée l'activité cérébrale de l'individu.

De telles interfaces ont tout d'abord été développées dans le but de fournir un canal de communication à des personnes ne pouvant plus effectuer d'activité motrice en leur apportant la possibilité de contrôler un effecteur grâce à leur activité cérébrale. On parle alors d'ICM active.

Depuis peu, ces interfaces ont été détournées de leur but premier afin de fournir un outil d'estimation de l'état mental d'une personne. On parle ainsi d'ICM passive pour nommer des systèmes qui n'utilisent pas une activité cérébrale volontairement dirigée vers le contrôle d'un effecteur, mais qui, en revanche, utilisent l'activité cérébrale d'une personne pour enrichir la communication homme-machine de manière implicite, par exemple pour servir d'indicateur d'état mental à des fins d'évaluation de conditions de sécurité, notamment lors de situations de pilotage ou de contrôle aérien.

L'activité oculaire d'une personne peut ainsi être mesurée pour fournir une estimation de son état mental, notamment pour en caractériser le niveau de fatigue mentale ou de charge mentale. Cette caractérisation peut être utilisée aussi bien dans une ICM passive que dans une ICM active où elle permet par exemple d'invalider des commandes lorsque l'état de fatigue est trop élevé, évitant ainsi des commandes erronées.

Il est en effet connu que différentes caractéristiques de l'activité oculaire sont modulées par ces états de fatigue ou de charge, ces deux phénomènes modifiant le taux de clignements (« Blink Rate » ou « Eye blink rate », BR), mais aussi la durée de ces clignements (« Blink Duration », BD) et la latence de ces clignements (« Blink Latency », BL), c'est-à-dire la durée entre l'apparition d'un stimulus et l'apparition d'un clignement. Ainsi, une augmentation de la charge mentale d'un individu a pour conséquence de diminuer le taux BR et la durée BD, mais aussi d'augmenter la latence BL. En revanche, une augmentation de la fatigue mentale d'un individu est reflétée par une augmentation du taux BR et de la durée BD. D'autres mesures fines de ces clignements, telles que des mesures d'amplitude, de vitesse d'ouverture, de fermeture (vitesse maximale ou moyenne), de durée de 50% de l'amplitude du clignement, de durée de 80% de l'amplitude du clignement (perclos 80), ainsi que les temps d'ouverture et de fermeture, peuvent également être utilisées pour caractériser l'état de fatigue ou de charge.

L'activité oculaire est classiquement mesurée au moyen d'électrodes positionnées sur le visage d'une personne afin d'enregistrer l'activité électro-oculographique (EOG). Il en résulte en pratique des désagréments (inconfortable pour les sujets et réduction de leur champ visuel).

Une piste possible pour contourner ces désagréments consiste à réaliser une mesure indirecte de l'activité oculaire d'une personne via des signaux EEG délivrés par un ensemble d'électrodes positionnées sur le cuir chevelu de la personne, signaux EEG dont on sait qu'ils sont extrêmement perturbés par différents types d'activités extra-cérébrales (oculaires, musculaires, cardiaques, etc.), appelées artéfacts.

On connaît ainsi des méthodes de détection automatique des clignements oculaires par le biais d'un signal EEG seul, sans utiliser de signal de référence comme l'EOG. On pourra à ce titre se reporter à l'article de L. Shoker et al. intitulé « Artifact removal from electroencephalograms using a Hybrid BSS-SVM algorithm », IEEE Signal Process. Lett., 12(10), pp. 721-724, 2005. Ces méthodes comprennent généralement une décomposition du signal EEG, généralement par séparation aveugle de sources. Ces méthodes extraient ensuite des caractéristiques sur le signal de ces sources et déterminent si celles-ci sont artéfactuelles ou non à l'aide de méthodes statistiques. Leur but ultime est la reconstruction du signal EEG débruité des sources artéfactuelles. Ces méthodes ont donc pour but la suppression du signal généré par les clignements oculaires, et ne visent donc pas à utiliser cette information afin de caractériser l'état mental de la personne.

Si quelques cas d'utilisation de mesures indirectes de l'activité oculaire par EEG à des fins d'utilisation et non uniquement de nettoyage ou de rejet ont pu être proposés (voir par exemple l'article de G. Borghini et al. intitulé « Assessment of mental fatigue during car driving by using high resolution EEG activity and neurophysiologic indices », Conf. Proc. IEEE Eng. Med. Biol. Soc. 2012;2012:6442-5), les méthodes utilisées ne sont pas détaillées et l'analyse des clignements reste assez basique en ce qu'elle ne consiste qu'en un simple comptage.

Or une caractérisation fine de ces clignements (par exemple en termes de durée et d'amplitude) est nécessaire dans certaines applications, notamment pour les applications d'ICM passives, comme par exemple des systèmes de surveillance de l'état mental d'un opérateur en situation à risque.

On connait du document de Zhiyu Qian et al. intitulé « Analysis of Fatigue with 3D TV based on EEG », 2013 International Conference on Orange Technologies (ICOT), 12 mars 2013, pages 306-309, un procédé de caractérisation de clignement oculaire.

## EXPOSÉ DE L'INVENTION

L'invention a pour objectif de répondre à ce besoin d'une analyse fine de clignements oculaires basée sur l'utilisation de signaux EEG, sans autre signal périphérique. Elle propose à cet effet un procédé selon la revendication 1.

- Certains aspects préférés mais non limitatifs de ce procédé sont définis dans les revendications dépendantes 2 à 11.
- L'invention porte également sur un système de caractérisation de l'activité oculaire d'une personne selon la revendication 12, ainsi que sur un produit programme d'ordinateur selon la revendication 13.

## BRÈVE DESCRIPTION DES DESSINS

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :

- la figure 1 illustre le positionnement de 11 électrodes sur le cuir chevelu dont le signal est enregistré et traité dans un exemple de réalisation de l'invention ;
- les figures 2 à 4 illustrent les performances de l'invention par comparaison d'un signal EOG vertical avec un signal EEGart reconstruit conformément à un mode de réalisation possible de l'invention.

## EXPOSÉ DÉTAILLÉ DE MODES DE RÉALISATION PARTICULIERS

L'invention porte selon un premier aspect sur un procédé de caractérisation de l'activité oculaire d'une personne à partir d'au moins deux signaux électro-encéphalographiques, chaque signal étant délivré par une électrode positionnée sur le cuir chevelu de la personne.

Les électrodes font typiquement partie d'un ensemble d'électrodes placées sur le cuir chevelu, par exemple un ensemble de 128 électrodes agencées selon la nomenclature 10-20.

Les perturbations présentes dans les signaux électro-encéphalographiques EEG délivrés par les différentes électrodes sont appelées artéfacts. Les artéfacts correspondent en grande partie à des activités électrophysiologiques non cérébrales d'origine corporelle enregistrés par l'EEG, comme des mouvements oculaires, des clignements, des activités musculaires, des activités cardiaques, etc. Les clignements, qui correspondent aux mouvements mécaniques de la paupière, génèrent des artéfacts qui apparaissent principalement sur les électrodes de la zone frontale.

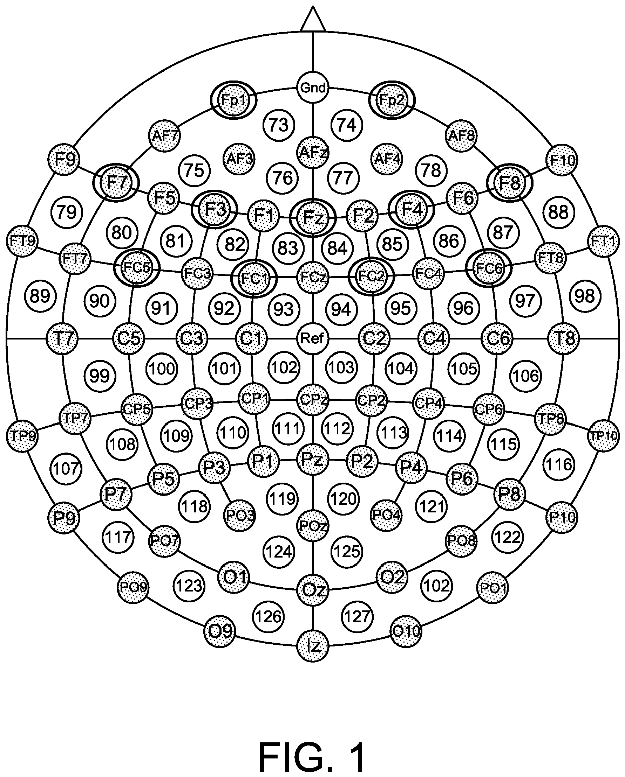

Dans le cadre de l'invention, les signaux d'une ou plusieurs des électrodes sont exploités pour caractériser l'activité oculaire, par exemple les signaux de 11 électrodes frontales où l'effet des clignements est le plus marqué. La figure 1 illustre à ce propos un positionnement possible de des électrodes sur le cuir chevelu dans la nomenclature 10-20, les 11 électrodes encerclées (électrodes Fp1, Fp2, F7, F3, Fz, F4, F8, FC5, FC1, FC2 et FC6) étant celles dont le signal est enregistré et traité dans cet exemple de réalisation de l'invention où l'électrode de référence est FCz, et l'électrode de masse est AFz.

Le procédé comprend une première étape d'acquisition du ou des signaux EEG (11 signaux dans l'exemple), par exemple sur un segment temporel de 20s. Considérant une fréquence d'échantillonnage de 500Hz, cette acquisition permet de former une matrice X de signaux EEG composé de M=11 lignes et de N=10000 colonnes. La $i^{\text{ème}}$ ligne de la matrice X correspond aux différents échantillons d'un signal EEG enregistrés par une électrode pendant 20 secondes. La $j^{\text{ème}}$ colonne de la matrice X correspond à la mesure à un instant d'échantillonnage donné des signaux des 11 électrodes. Ainsi, par signal EEG acquis, on entend dans le cadre de l'invention un ensemble d'échantillons sur un bloc temporel. L'invention est mise en oeuvre sur un bloc temporel donné, et peut bien évidemment être répétée sur d'autres bloc temporels.

Dans un mode de réalisation, le procédé peut comprendre un filtrage des signaux EEG acquis, typiquement un filtrage permettant d'éliminer la ligne de base (composante continue) et les composantes hautes fréquences qui n'apportent pas d'information pertinente. Un filtrage passe bande à basses fréquences, par exemple un filtrage laissant passer la bande 0,5Hz-40Hz, peut pour cela être mis en oeuvre. On utilise par exemple un filtre à réponse impulsionnelle infinie de type Butterworth d'ordre 5.

Le procédé selon l'invention comporte une étape de séparation du ou des signaux EEG acquis, le cas échéant filtrés comme indiqué ci-dessus, en une pluralité de sources d'origine.

3

Un signal EEG est ainsi modélisé comme un mélange de composantes correspondant chacune à une source d'origine cérébrale ou à une source d'origine artéfactuelle. On suppose que les différentes sources (d'origine cérébrale ou non cérébrale) sont additionnées par la conduction électrique et récupérées par les électrodes, de sorte que chaque échantillon $x_i$ d'un signal EEG délivré par une électrode i est un mélange linéaire instantané de plusieurs sources d'origine, mélange caractérisé par une matrice de mélange notée $W^{-1}$.

Cette séparation en sources d'origine est par exemple une séparation aveugle de sources (SAS - ou BSS pour « blind source séparation » en anglais) qui permet d'extraire des sources indépendantes à partir de signaux mesurés.

En d'autres termes, cette séparation permet d'estimer une matrice de sources Z et une matrice de séparation W telles que Z=W*Xf, où Xf correspond à la matrice des signaux EEG acquis et le cas échéant filtrés. On a ainsi Xf=$W^{-1}$*Z où la matrice inverse de la matrice de séparation W (matrice de mélange $W^{-1}$) illustre la contribution de chacune des sources à la formation de chacun des signaux, c'est-à-dire le poids de chacune des composantes du signal EEG correspondant à une source d'origine.

$$
\begin{bmatrix} Xf(1,1) & \cdots & Xf(1,T) \\ \vdots & \ddots & \vdots \\ Xf(N,1) & \cdots & Xf(N,T) \end{bmatrix} = \begin{bmatrix} W^{-1}(1,1) & \cdots & W^{-1}(1,J) \\ \vdots & \ddots & \vdots \\ W^{-1}(N,1) & \cdots & W^{-1}(N,J) \end{bmatrix} * \begin{bmatrix} Z(1,1) & \cdots & Z(1,T) \\ \vdots & \ddots & \vdots \\ Z(J,1) & \cdots & Z(J,T) \end{bmatrix}
$$

Avec :

$X_f(i,j)$ = signal, éventuellement filtré, mesuré par l'électrode i à l'instant j, N et T désignant respectivement le nombre d'électrodes mises en oeuvre et le nombre d'échantillons temporels considérés.

$W^{-1}(i,k)$ = coefficient de pondération correspondant au couple source k-électrode i, K désignant le nombre de sources considérées.

$Z(k,j)$ = valeur du signal de source k à l'échantillon temporel j.

Ainsi, chaque ligne i de la matrice Xf correspond à un signal $x_i$ mesuré par une électrode i, après échantillonnage temporel.

Dans un mode de réalisation, on considère que la matrice Z est de même dimension que la matrice Xf, le nombre de sources d'origine étant ainsi égal au nombre de voies de mesure (soit 11 dans l'exemple). Autrement dit, dans ce mode de réalisation, N = J. Naturellement, dans d'autres modes de réalisation, J peut être supérieur ou inférieur à N.

La séparation aveugle de source est par exemple réalisée selon l'algorithme SOBI (« Second Order Blind Identification ») en supposant pour cela que les sources sont stationnaires et décorrélées quel que soit le décalage. La solution s'appuyant sur cette hypothèse est la décorrélation simultanée de signaux pour différents décalages et donc la diagonalisation simultanée des différentes matrices d'auto-corrélation.

L'estimation de la matrice de séparation W est ainsi réalisée en deux étapes. Au cours de la première étape, une matrice de passage Q est calculée afin de décorréler les signaux EEG (par diagonalisation de la matrice de covariance). Dans une deuxième étape, une matrice de rotation J est calculée afin d'obtenir des sources décorrélées indépendamment du décalage temporel. Cette matrice de rotation J est obtenue par diagonalisation conjointe de 10 matrices de corrélation calculées avec des décalages allant de 1 à 10 périodes d'échantillonnage. On obtient au final la matrice de séparation W=J*Q.

Le procédé comporte ensuite une étape d'identification d'une source d'origine artéfactuelle oculaire parmi les différentes sources séparées par SAS.

Cette identification repose par exemple sur une classification supervisée permettant de classer chacune des sources d'origine séparées dans une classe artéfactuelle ou dans une classe non-artéfactuelle. Elle est basée sur une classification construite lors d'une phase d'apprentissage, et vise à attribuer un vecteur de caractéristiques Vj d'une source à la classe artéfactuelle ou à la classe non-artéfactuelle. La dimension, notée c, de ce vecteur de caractéristiques correspond au nombre de caractéristiques retenues pour établir la classification. Dans cet exemple, c = 6.

Pour un vecteur caractéristique Vj, sa probabilité (*a posteriori*) d'appartenance à la classe artéfactuelle s'écrit selon la loi de Bayes : $P(SA/V_j) = \dfrac{p(V_j / SA)}{p(V_j / SA) + p(V_j / SNA)}$, tandis que sa probabilité d'appartenance à la classe non-artefactuelle s'écrit selon la loi de Bayes : $P(SNA/V_j) = \dfrac{p(V_j / SNA)}{p(V_j / SA) + p(V_j / SNA)}$, où *p(Vj/SA)* est la densité de probabilité du vecteur de caractéristiques Vj au sein de la classe artéfactuelle, et *p(Vj/SNA)* est la densité de probabilité du vecteur de caractéristiques Vj au sein de la classe non artéfactuelle. On considère ici que la probabilité a priori de chacune des classes est identique : *p(SA) = p(SNA)*.

Les densités de probabilité des vecteurs de caractéristiques p(Vj/SA) et p(Vj/SNA) suivent chacune une loi de probabilité gaussienne pour la classe concernée. On considère ainsi une classe artéfactuelle gaussienne de moyenne $m_{SA}$ et de matrice de covariance $\Sigma_{SA}$, et une classe non artéfactuelle gaussienne de moyenne $m_{SNA}$ et de matrice de covariance $\Sigma_{SNA}$. Ces moyennes et matrices de covariance sont déterminées lors de la phase d'apprentissage.

Les densités de probabilité s'expriment dès lors ainsi :

$$p(V_j / SA) = \frac{1}{(2\pi)^{c/2}(\det \Sigma_{SA})^{1/2}} \exp(-\frac{1}{2}(V_j - m_{SA})\Sigma_{SA}^{-1}(V_j - m_{SA})^T)$$

$$p(V_j / SNA) = \frac{1}{(2\pi)^{c/2}(\det \Sigma_{SNA})^{1/2}} \exp(-\frac{1}{2}(V_j - m_{SNA})\Sigma_{SNA}^{-1}(V_j - m_{SNA})^T)$$

Pour chacune des sources d'origine séparées Sj, on vient ainsi calculer un vecteur de caractéristiques Vj, puis sa densité de probabilité *p(SA/Vj)* et sa densité de probabilité *p(SNA/Vj)* pour en déduire sa probabilité d'appartenance à une classe artéfactuelle ou à une classe non artéfactuelle.

Plus particulièrement, et comme présenté ci-dessus, connaissant le vecteur caractéristique Vj d'une source d'origine séparée, la densité de probabilité d'appartenance à la classe artéfactuelle *p(SA/Vj),* respectivement à la classe non artéfactuelle *p(SNA/Vj),* de la source d'origine séparée est calculée selon la loi de Bayes en utilisant une loi de probabilité gaussienne *p(Vj/SA)* pour un vecteur de caractéristiques d'une source de la classe artéfactuelle et une loi de probabilité gaussienne *p(Vj/ SNA)* pour un vecteur de caractéristiques d'une source de la classe non artéfactuelle. Les probabilités a priori d'appartenance à la classe artéfactuelle P(SA) et non artéfactuelle P(SNA) sont supposées égales.

La phase d'apprentissage pour l'estimation des moyennes et matrices de covariance des classes gaussiennes (artéfactuelle et non artéfactuelle) est par exemple la suivante. Les signaux EEG d'une personne sont enregistrés, par exemple pendant 15 à 20 minutes, et découpés en segments temporels, par exemple de 20 secondes. Pour chaque segment temporel, on réalise une séparation de sources selon l'algorithme SOBI, et on réalise une inspection visuelle des sources séparées pour identifier des sources artéfactuelles. Une base d'apprentissage constituée d'environ 50 sources artéfactuelles et 500 sources non artéfactuelles est ainsi créée. Pour chaque source de la base d'apprentissage, on vient ensuite calculer un vecteur de caractéristiques (tel que par exemple détaillé ci-dessous, et en tout état de cause identique à celui calculé pour le calcul des probabilités a posteriori d'appartenance à la classe artéfactuelle et à la classe non-artéfactuelle). Une élimination des sources présentant un vecteur de caractéristiques aberrant, en ce sens qu'il présente une caractéristique qui s'écarte significativement des caractéristiques correspondantes des vecteurs caractéristiques des sources appartenant à la même catégorie (artéfactuelle ou non), peut être réalisée.

Pour chaque source, et pour chaque caractéristique du vecteur de caractéristiques, on procède par exemple au calcul de la distance moyenne de chaque caractéristique à ses cinq plus proches voisins dans la classe, puis au calcul de la moyenne des distances moyennes au sein de la classe. On supprime alors les sources présentant une caractéristique dont la distance moyenne à ses cinq plus proches voisins est supérieure à cinq fois la moyenne de la classe. On calcule pour finir la moyenne (mSA, mSNA) et la matrice de covariance ($\Sigma_{SNA}$, $\Sigma_{SA}$) de la classe à partir des sources restantes.

Dans le cadre de l'invention, le vecteur de caractéristiques d'une source comporte une caractéristique temporelle et/ou une caractéristique spatiale et/ou une caractéristique fréquentielle et/ou d'une caractéristique d'amplitude de la source.

Une caractéristique temporelle de la source correspond à l'évolution des différents échantillons formant une ligne de la matrice de sources Z. A titre d'exemple, on peut caractériser temporellement une source en calculant son coefficient d'aplatissement, ou Kurtosis, qui permet de vérifier si les échantillons de la source vérifient une distribution gaussienne. On considère en effet qu'une source artéfactuelle oculaire ne présente pas une telle distribution gaussienne.

Le Kurtosis Kj de la source j est donné par $K_j = \dfrac{\frac{1}{N}\sum_{k=1}^{N}(Z(j,k) - m_j)^4}{(\frac{1}{N}\sum_{k=1}^{N}(Z(j,k) - m_j)^2)^2}$ avec $m_j = \dfrac{1}{N}\sum_{k=1}^{N}Z(j,k)$.

On peut également caractériser temporellement une source en calculant son coefficient de dissymétrie, ou *skewness* en anglais. Une gaussienne étant symétrique (coefficient nul), on considère qu'une source artéfactuelle oculaire présente un coefficient éloigné de zéro. Le coefficient de dissymétrie SKj de la source j est donné par

$$Sk_j = \frac{\dfrac{1}{N}\sum\limits_{k=1}^{N}(Z(j,k)-m_j)^3}{(\dfrac{1}{N}\sum\limits_{k=1}^{N}(Z(j,k)-m_j)^2)^{1.5}}.$$

Une caractéristique spatiale d'une source a trait à la manière dont la source impacte spatialement le cuir chevelu, plus particulièrement au niveau des électrodes délivrant les signaux EEG utilisés dans le cadre de l'invention.

La forte influence d'une source sur les signaux mesurés se traduit par une valeur absolue importante des coefficients qui correspondent à cette source dans la matrice de mélange $W^{-1}$. Par conséquent, la puissance et la propagation spatiale de chaque source peuvent être évaluées à travers la matrice de mélange $W^{-1}$, plus particulièrement par les éléments de la colonne correspondante de la matrice de mélange $W^{-1}$. En ce qui concerne la source artéfactuelle oculaire, la colonne correspondante devrait avoir des valeurs significativement plus élevées (car cette source est très présente et en général présente une forte amplitude) et plus contrastées (car plus distribuées, le signal artéfactuel oculaire se retrouvant essentiellement dans les deux électrodes positionnées directement au-dessus des yeux).

On peut ainsi caractériser spatialement une source j en évaluant sa contribution dans le mélange. On calcule pour cela sa dispersion Dj qui correspond à la moyenne des valeurs absolues des coefficients de la colonne j de la matrice de mélange W-1 selon $D_j = \sum\limits_{l=1}^{M}\left|W^{-1}(l,j)\right|$. On peut également caractériser spatialement une source j en déterminant sa propagation Pj pour évaluer si elle se projette d'une manière pseudo-similaire sur toutes les électrodes (en d'autres termes on vérifie le degré d'homogénéité de l'impact de la source sur les électrodes) :

$$P_j = \frac{\dfrac{1}{M-1}\sum\limits_{l=1}^{M}(\left|W^{-1}(l,j)\right|-mw_j)^2}{Dj} \text{ avec } mw_j = \frac{1}{M}\sum\limits_{l=1}^{M}\left|W^{-1}(l,j)\right|.$$

Le recours à ces deux paramètres Dj, PJ est décrit dans la publication de R. Romo Vázquez, et al., « Blind source separation, wavelet denoising and discriminant analysis for EEG artefacts and noise cancelling. Biomed ». Signal Process. Control (2011).

Une caractéristique fréquentielle d'une source repose sur un calcul de la densité de puissance spectrale DSPj de la source j, par exemple à l'aide du périodogramme moyenné de Welch calculé sur des fenêtres temporelles de 1025 échantillons (environ 2 secondes) avec recouvrement de 512 échantillons.

On peut ainsi caractériser en fréquence une source en venant calculer une proportion de composantes basses fréquences, par exemple le pourcentage PBFj de composantes basses fréquences dans la gamme 0,5-5Hz sur l'ensemble de la gamme 0,5-40Hz : $P_{BF\,j} = \dfrac{\int_{0.5}^{5} DSP_j(f)df}{\int_{0.5}^{40} DSP_j(f)df}.$

Une caractéristique d'amplitude de la source correspond par exemple à la distribution en amplitude de la source. On peut ainsi venir calculer un ratio entre les amplitudes d'un premier et d'un deuxième percentiles d'échantillons de la source, par exemple le ratio $R_{99/50j}$ entre le 99ème percentile Per99(j) (valeur en-dessous de laquelle se trouvent 99% des échantillons, en valeur absolue, de la ligne j de la matrice de sources Z) et le 50ème percentile Per50(j), (valeur en dessous de laquelle se trouvent 50% des échantillons en valeur absolue, de la ligne j de la matrice de sources Z) :

$$R_{99/50j} = \frac{Per_{99}(j)}{Per_{50}(j)}.$$

Dans un mode de réalisation de l'invention, le vecteur caractéristique Vj d'une source est constitué des six caractéristiques exemplifiées ci-dessus : $V_j = \lfloor K_j\ Sk_j\ D_j\ P_j\ P_{BFj}\ R_{99/50j}\rfloor$.

On a vu précédemment, que pour chacune des sources d'origine séparées Sj, on vient évaluer sa densité de probabilité $p(SA/Vj)$ d'appartenance à la classe artéfactuelle et sa densité de probabilité $p(SNA/Vj)$ d'appartenance à la classe non artéfactuelle.

Dans un mode de réalisation possible, une source séparée est identifiée comme étant une source d'origine artéfactuelle lorsque sa probabilité $p(SA/Vj)$ d'appartenance à la classe artéfactuelle est supérieure à sa probabilité $p(SNA/Vj)$ d'appartenance à la classe non artéfactuelle. Lorsqu'aucune source n'est identifiée comme artéfactuelle, aucun clignement n'est identifié sur le segment temporel considéré. Si plusieurs sources sont identifiées comme étant artéfactuelles, celle dont la probabilité d'appartenance à la classe artéfactuelle est la plus importante peut être retenue et identifiée comme étant la source artéfactuelle d'origine oculaire principale.

Lorsqu'une source artéfactuelle oculaire est identifiée sur le segment temporel considéré, le procédé selon l'invention comporte une étape de reconstruction d'au moins un signal électro-encéphalographique correspondant à la source d'origine artéfactuelle oculaire identifiée. En d'autres termes, on vient reconstruire le signal artéfactuel (source artéfactuelle oculaire) dans l'espace des capteurs (électrodes). Le signal EEG reconstruit reflète ainsi uniquement l'activité oculaire.

Pour ce faire, le procédé comprend l'application de la matrice de mélange $W^{-1}$ à la matrice de sources d'origine dans laquelle toutes les sources d'origine, à l'exception de la source artéfactuelle oculaire identifiée, sont annulées.

Soit $Z_0 = [0\ 0\ ...\ 0]$, un vecteur de dimension 1 ligne et T colonnes, et $Z_q = \begin{bmatrix} Z_0 \\ Z_0 \\ Z_q \\ Z_0 \end{bmatrix}$ une matrice de même dimension que la matrice de sources Z dont toutes les lignes, excepté la $q^{ème}$ corespondant à la source artéfactuelle oculaire identifiée, ont été remplacées par des zéros. La reconstruction de la composante $X_q$ de l'au moins un signal EEG ayant pour source d'origine la source artéfactuelle oculaire identifiée s'écrit alors $X_q = W^{-1}Z_q$.

Selon une variante, le procédé comporte la sélection de plusieurs sources artéfactuelles, par exemple s sources artéfactuelles, dont les probabilités respectives d'appartenance à la classe artéfactuelle sont les plus élevées. La reconstruction du signal EEG est alors effectuée à partir des sources ainsi sélectionnées, les autres sources, non sélectionnées, étant annulées.

On comprend alors que la reconstruction du signal électro-encéphalographique de tout ou partie des électrodes i est réalisée principalement, voire uniquement à l'aide des sources considérées comme artéfactuelles oculaires, ou de la source considérée comme artéfactuelle oculaire principale. Durant cette reconstruction, la contribution des sources considérées comme non artéfactuelles est annulée. Autrement dit, la reconstruction du signal électro-encéphalographique est effectuée en considérant uniquement la contribution des sources (ou de la source) considérées comme artéfactuelles.

Le procédé comprend ensuite une étape de détermination d'informations relatives à un clignement oculaire à partir de la reconstruction $X_q$ de signaux EEG, la notation $X_q$ désignant la matrice rassemblant les signaux électro-encéphalographiques reconstruits à partir de la $q_{ième}$ source.

On relèvera que le retour dans l'espace des capteurs, par la reconstruction du signal artéfactuel (source artéfactuelle oculaire) dans l'espace des capteurs (électrodes), permet une caractérisation fine de l'activité oculaire.

La matrice $X_q$ comporte au moins deux signaux $x_q^i$, correspondant chacun à une électrode i.

Dans un mode de réalisation, lorsque le procédé exploite des signaux EEG délivrés par au moins deux électrodes, cette détermination peut n'être réalisée que sur la base d'un seul signal EEG reconstruit, noté EEGart. Dans ce cas, on peut conserver, parmi les différents signaux $x_q^i$, celui qui présente l'amplitude maximum la plus importante.

Le signal EEGart peut être filtré de manière similaire aux signaux EEG d'origine, par exemple dans la bande 0,5-10Hz au moyen d'un filtre de Butterworth d'ordre 5.

Dans un mode de réalisation, on réalise un seuillage en amplitude adaptatif du signal électro-encéphalographique EEGart reconstruit et retenu pour la détermination d'informations relatives à un clignement oculaire. Ce seuillage adaptatif est par exemple de la forme seuil = medi + $\alpha$ * $\delta$, où medi désigne la médiane des échantillons de EEGart, $\alpha$ est un paramètre par exemple fixé à 10, et $\delta$ = $mediane(|EEG_{art}\text{-}medi|)$.

Le procédé comprend ensuite une sélection des échantillons du signal EEGart supérieurs au seuil et qui correspondent aux portions les plus amples des clignements. On constitue de telle manière des fenêtres temporelles de caractérisation du clignement, le seuillage fournissant un instant de début tdeb et un instant de fin tfin au cours duquel le signal EEGart se situe au-dessus du seuil.

Le clignement peut être caractérisé en recherchant son instant de début et de fin. Le signal EEGart est dérivé numériquement par une simple différence entre deux échantillons consécutifs : dEEGart(k)= EEGart(k)- EEGart(k-1). Le début du clignement (début) est par exemple localisé comme le dernier instant où la dérivée du signal EEGart s'annule sur l'intervalle de temps [tdeb-250ms, tdeb]. La fin du clignement (fin) correspond par exemple au premier instant où la dérivée s'annule sur l'intervalle de temps [tfin, tfin+250ms]. Le clignement peut également être caractérisé en déterminant l'instant de fermeture (tf) de la paupière, par exemple comme étant le premier instant où la dérivée s'annule sur l'intervalle [tdeb, tfin].

L'une et/ou l'autre des caractéristiques suivantes, classiquement utilisées pour estimer l'état mental d'une personne, peuvent également être calculées :

- Amplitude du clignement: valeur maximale mesurée entre début et fin ;
- Durée du clignement : temps écoulé entre début et fin ;
- Durée 50 : temps écoulé entre le début et la fin de 50% d'amplitude du clignement ;
- Perclos 80: temps écoulé entre le début et la fin de 80% d'amplitude du clignement ;
- Temps de fermeture : temps écoulé entre début et tf ;
- Temps d'ouverture: temps écoulé entre tf et fin ;
- Vitesse moyenne d'ouverture : moyenne de la dérivée entre début et tf ;
- Vitesse moyenne de fermeture : moyenne de la dérivée entre tf et fi ;
- Vitesse maximale d'ouverture : valeur maximale mesurée sur la dérivée entre début et tf.

L'invention n'est pas limitée au procédé tel que précédemment décrit mais s'étend également à un système de caractérisation de l'activité oculaire d'une personne, comprenant des moyens de traitement configurés pour :

- séparer au moins deux signaux électro-encéphalographiques de la personne en une pluralité de sources d'origine, lesdits signaux étant modélisés comme un mélange de composantes correspondant chacune à une source d'origine cérébrale ou à une source d'origine artéfactuelle ;
- identifier et sélectionner au moins une source d'origine artéfactuelle oculaire parmi les sources séparées,

caractérisé en ce que les moyens de traitement sont en outre configurés pour reconstruire un signal électro-encéphalographique correspondant à ladite au moins une source d'origine artéfactuelle oculaire sélectionnée.

Et l'invention s'étend également à un produit programme d'ordinateur comprenant des instructions de code pour l'exécution des étapes de séparation, d'identification/sélection et de reconstruction du procédé décrit précédemment, lorsque ledit programme est exécuté sur un ordinateur. Ledit programme peut en outre réaliser la détermination des informations relatives à un clignement oculaire à partir du signal EEG reconstruit correspondant à ladite au moins une source d'origine artéfactuelle oculaire sélectionnée.

On relèvera qu'une évaluation des performances de l'invention a été réalisée qui a consisté à comparer un signal électro-oculographique vertical (EOVG) au signal EEGart décrit précédemment. Ces performances s'avèrent très bonnes en ce qu'elles affichent un taux de détection correcte des clignements supérieur à 90% et une corrélation moyenne dépassant les 80% entre les caractéristiques extraites de l'EOGV et celles de l'EEGart.

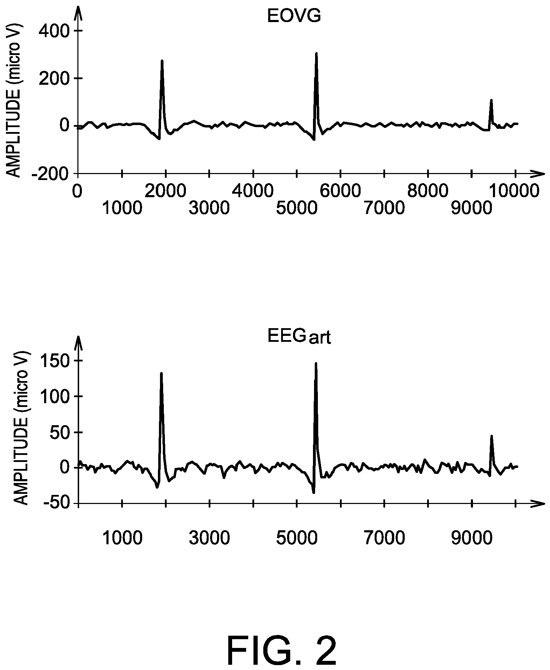

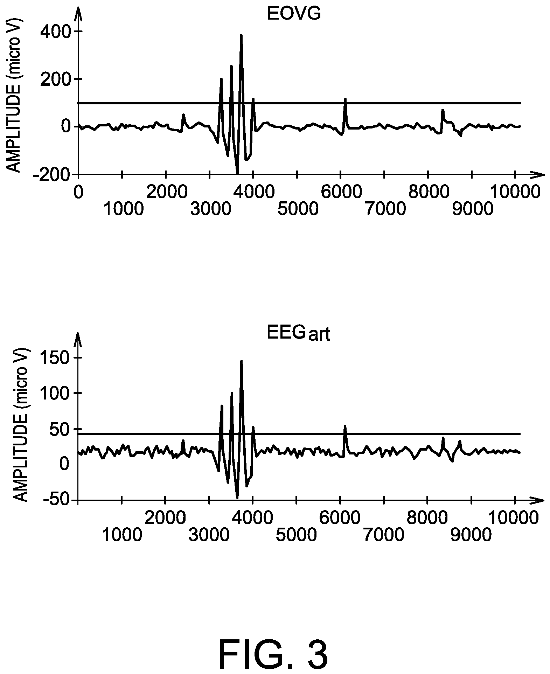

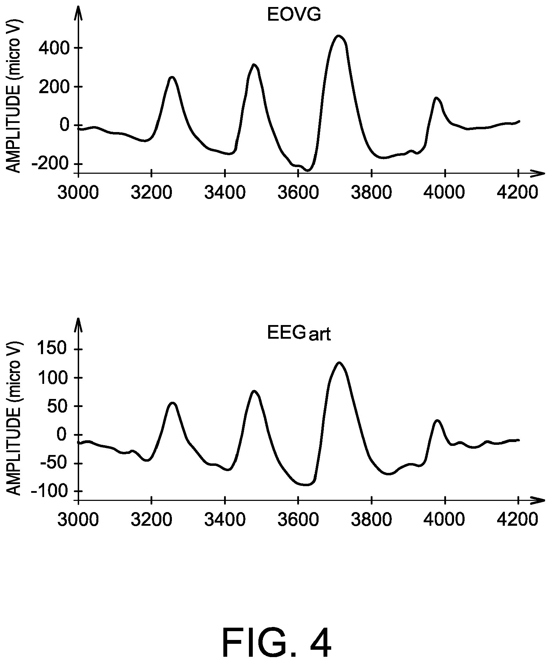

A cet égard, la figure 2 illustre le signal EOVG (en haut) et le signal reconstruit EEGart (en bas) sur un bloc temporel de 20 s comprenant 10 000 échantillons. La figure 3 illustre le seuillage en amplitude de ces signaux pour sélectionner des échantillons de ces signaux supérieurs au seuil et qui correspondent aux portions les plus amples des clignements. La figure 4 illustre une fenêtre temporelle de caractérisation du clignement ainsi formée, ici constituée des échantillons 3000 à 4200, et sur laquelle on constate la très bonne corrélation des signaux EOGV et EEGart.

## Revendications

1. Procédé de caractérisation de l'activité oculaire d'une personne à partir d'au moins deux signaux électro-encéphalographiques délivrés par des électrodes positionnées sur le cuir chevelu de la personne, comprenant les étapes de :

   - séparation des signaux électro-encéphalographiques en une pluralité de sources d'origine, lesdits signaux étant modélisés comme un mélange de composantes correspondant chacune à une source d'origine cérébrale ou à une source d'origine artéfactuelle, ladite séparation comprenant la détermination d'une matrice de mélange reliant une matrice de signaux électro-encéphalographiques à une matrice de sources d'origine ;
   - identification et sélection d'au moins une source d'origine artéfactuelle oculaire parmi les sources séparées,
   - reconstruction d'au moins un signal électro-encéphalographique correspondant à ladite au moins une source d'origine artéfactuelle oculaire sélectionnée, ladite reconstruction comprenant l'application de la matrice de mélange à la matrice de sources d'origine dans laquelle toutes les sources d'origine, à l'exception de ladite au moins une source artéfactuelle oculaire sélectionnée, sont annulées ;
   - détermination d'informations relatives à un clignement oculaire à partir dudit signal électro-encéphalographique reconstruit.

2. Procédé selon la revendication 1, dans lequel l'identification de la source d'origine artéfactuelle oculaire comprend le calcul, pour chacune des sources d'origine séparées, d'une probabilité d'appartenance à une classe artéfactuelle et/ou d'une probabilité d'appartenance à une classe non artéfactuelle.

3. Procédé selon la revendication 2, comprenant la détermination d'un vecteur de caractéristiques pour chaque source d'origine séparée, le calcul d'une probabilité d'appartenance à une classe artéfactuelle et/ou d'une probabilité d'appartenance à une classe non artéfactuelle étant obtenu en appliquant une densité de probabilité, préalablement établie, audit vecteur de caractéristiques.

4. Procédé selon l'une des revendications 2 et 3, comprenant l'identification d'une source d'origine séparée comme étant une source d'origine artéfactuelle en fonction de sa probabilité d'appartenance à la classe artéfactuelle ou en fonction de sa probabilité d'appartenance à la classe non artéfactuelle.

5. Procédé selon l'une des revendications 1 à 4, dans lequel l'identification de la source artéfactuelle oculaire comprend le calcul d'une caractéristique temporelle et/ou d'une caractéristique spatiale et/ou d'une caractéristique fréquentielle et/ou d'une caractéristique d'amplitude de chacune des sources d'origine séparées.

6. Procédé selon la revendication 5, comprenant le calcul d'une caractéristique temporelle du type coefficient d'aplatissement ou du type coefficient de dissymétrie et/ou une caractéristique spatiale correspondant à un impact et à un degré d'homogénéité de l'impact de la source sur le cuir chevelu de la personne et/ou un caractéristique fréquentielle correspondant à une proportion de composantes basses fréquences et/ou une caractéristique d'amplitude correspondant à un ratio entre les échantillons d'un premier et un deuxième percentiles.

7. Procédé selon l'une des revendications 1 à 6, dans lequel au moins deux signaux électro-encéphalographiques sont reconstruits, et dans lequel on retient pour la détermination d'informations relatives à un clignement oculaire le signal reconstruit présentant le maximum le plus important.

8. Procédé selon l'une des revendications 1 à 7, comprenant un seuillage en amplitude adaptatif du signal électro-encéphalographique reconstruit retenu pour la détermination d'informations relatives à un clignement oculaire.

9. Procédé selon l'une des revendications 1 à 8, dans lequel la détermination d'informations relatives à un clignement oculaire comprend le calcul de la dérivée du signal électro-encéphalographique reconstruit et la recherche d'instants où ladite dérivée s'annule.

10. Procédé selon l'une des revendications 1 à 9, dans lequel la détermination d'informations relatives à un clignement oculaire comprend la détermination d'un début de clignement, d'une fin de fermeture de la paupière et d'une fin de clignement, et dans lequel les informations relatives à un clignement oculaire comprennent la durée du clignement, l'amplitude maximale du clignement, le temps de fermeture de la paupière, le temps d'ouverture de la paupière.

11. Procédé selon la revendication 10, dans lequel les informations relatives à un clignement oculaire comprennent en outre la durée entre le début et la fin d'une fraction donnée de l'amplitude maximale du clignement et/ou une vitesse d'ouverture ou de fermeture de la paupière.

12. Système de caractérisation de l'activité oculaire d'une personne à partir d'au moins deux signaux électro-encéphalographiques délivrés par des électrodes positionnées sur le cuir chevelu de la personne, comprenant des moyens de traitement configurés pour :

- séparer au moins deux signaux électro-encéphalographiques de la personne en une pluralité de sources d'origine par détermination d'une matrice de mélange reliant une matrice de signaux électro-encéphalographiques à une matrice de sources d'origine, lesdits signaux étant modélisés comme un mélange de composantes correspondant chacune à une source d'origine cérébrale ou à une source d'origine artéfactuelle ;
- identifier et sélectionner au moins une source d'origine artéfactuelle oculaire parmi les sources séparées,
- reconstruire au moins un signal électro-encéphalographique correspondant à ladite au moins une source d'origine artéfactuelle oculaire identifiée en appliquant la matrice de mélange à la matrice de sources d'origine dans laquelle toutes les sources d'origine, à l'exception de ladite au moins une source artéfactuelle oculaire sélectionnée, sont annulées;

- déterminer des informations relatives à un clignement oculaire à partir dudit signal électro-encéphalographique reconstruit.

13. Produit programme d'ordinateur comprenant des instructions de code pour l'exécution des étapes du procédé selon l'une des revendications 1 à 11, lorsque ledit programme est exécuté sur un ordinateur.

**Patentansprüche**

1. Verfahren zur Charakterisierung der Augenaktivität einer Person ausgehend von wenigstens zwei elektroenzephalographischen Signalen, die durch Elektroden geliefert werden, die an der Kopfhaut der Person positioniert sind, umfassend die Schritte:

   - Separieren der elektroenzephalographischen Signale in eine Mehrzahl von Ursprungsquellen, wobei die Signale als eine Mischung von Komponenten modelliert werden, die jeweils einer zerebralen Ursprungsquelle oder einer artefaktuellen Ursprungsquelle entsprechen, wobei die Separation die Bestimmung einer Mischungsmatrix umfasst, die eine Matrix von elektroenzephalographischen Signalen mit einer Matrix von Ursprungsquellen verbindet;
   - Identifizieren und Auswählen wenigstens einer artefaktuellen Augenursprungsquelle aus den separierten Quellen,
   - Rekonstruieren wenigstens eines elektroenzephalographischen Signals, das der wenigstens einen ausgewählten artefaktuellen Augenursprungsquelle entspricht, wobei die Rekonstruktion die Anwendung der Mischungsmatrix auf die Matrix von Ursprungsquellen umfasst, bei der alle Ursprungsquellen mit Ausnahme der wenigstens einen ausgewählten artefaktuellen Augenquelle, annuliert sind;
   - Bestimmen von Informationen bezüglich eines Augenblinzelns ausgehend von dem rekonstruierten elektroenzephalographischen Signal.

2. Verfahren nach Anspruch 1, bei dem das Identifizieren der artefaktuellen Augenursprungsquelle die Berechnung, für jede der separierten Ursprungsquellen, einer Wahrscheinlichkeit einer Zugehörigkeit zu einer artefaktuellen Klasse und/oder einer Wahrscheinlichkeit einer Zugehörigkeit zu einer nicht-artefaktuellen Klasse umfasst.

3. Verfahren nach Anspruch 2, umfassend die Bestimmung eines Vektors von Eigenschaften für jede separierte Ursprungsquelle, wobei die Berechnung einer Wahrscheinlichkeit einer Zugehörigkeit zu einer artefaktuellen Klasse und/oder einer Wahrscheinlichkeit einer Zugehörigkeit zu einer nicht-artefaktuellen Klassen erhalten wird, indem man eine zuvor erstellte Wahrscheinlichkeitsdichte auf den Vektor von Eigenschaften anwendet.

4. Verfahren nach einem der Ansprüche 2 und 3, umfassend das Identifizieren einer separierten Ursprungsquelle als eine artefaktuelle Ursprungsquelle als Funktion ihrer Wahrscheinlichkeit einer Zugehörigkeit zu der artefaktuellen Klasse oder als Funktion ihrer Wahrscheinlichkeit einer Zugehörigkeit zur nicht-artefaktuellen Klasse.

5. Verfahren nach einem der Ansprüche 1 bis 4, bei dem das Identifizieren der artefaktuellen Augenquelle die Berechnung einer zeitlichen Eigenschaft und/oder einer räumlichen Eigenschaft und/oder einer Frequenzeigenschaft und/oder einer Amplitudeneigenschaft von jeder der separierten Ursprungsquellen umfasst.

6. Verfahren nach Anspruch 5, umfassend die Berechnung einer zeitlichen Eigenschaft vom Typ Abflachungskoeffizient oder von Typ Dissymmetriekoeffizient und/oder einer räumlichen Eigenschaft entsprechend einer Einwirkung und einem Homogenitätsgrad der Einwirkung der Quelle auf die Kopfhaut der Person und/oder einer Frequenzeigenschaft entsprechend einem Verhältnis von niederfrequenten Komponenten und/oder einer Amplitudeneigenschaft entsprechend einem Verhältnis zwischen den Abtastwerten eines ersten und eines zweiten Perzentils.

7. Verfahren nach einem der Ansprüche 1 bis 6, bei dem wenigstens zwei elektroenzephalographische Signale rekonstruiert werden, und bei dem man für die Bestimmung von Informationen bezüglich eines Augenblinzelns das rekonstruierte Signal behält, das das größte Maximum aufweist.

8. Verfahren nach einem der Ansprüche 1 bis 7, umfassend eine adaptive Schwellenbildung hinsichtlich der Amplitude des rekonstruierten elektroenzephalographischen Signals, das für die Bestimmung von Informationen bezüglich eines Augenblinzelns behalten wird.

**9.** Verfahren nach einem der Ansprüche 1 bis 8, bei dem die Bestimmung von Informationen bezüglich eines Augenblinzelns die Berechnung der Ableitung des rekonstruierten elektroenzephalographischen Signals und die Suche nach Zeitpunkten umfasst, wo die Ableitung verschwindet.

**10.** Verfahren nach einem der Ansprüche 1 bis 9, bei dem die Bestimmung von Informationen bezüglich eines Augenblinzelns die Bestimmung eines Anfangs des Blinzelns umfasst, eines Endes des Schließens des Augenlids und eines Ende des Blinzelns, und bei dem die Informationen bezüglich eines Augenblinzelns die Dauer des Blinzelns, die maximale Amplitude des Blinzelns, die Zeit des Schließens des Augenlids und die Zeit des Öffnens des Augenlids umfassen.

**11.** Verfahren nach Anspruch 10, bei dem die Informationen bezüglich eines Augenblinzelns ferner die Dauer zwischen dem Anfang und dem Ende eines gegebenen Bruchteils der maximalen Amplitude des Blinzelns und/oder eine Geschwindigkeit des Öffnens oder des Schließens des Augenlids umfassen.

**12.** System zur Charakterisierung der Augenaktivität einer Person ausgehend von wenigstens zwei elektroenzephalographischen Signalen, die durch Elektroden geliefert werden, die an der Kopfhaut der Person positioniert sind, umfassend Verarbeitungsmittel, die zu Folgendem ausgelegt sind:

- Separieren von wenigstens zwei elektroenzephalographischen Signalen der Person in eine Mehrzahl von Ursprungsquellen durch Bestimmung einer Mischungsmatrix, die eine Matrix von elektroenzephalographischen Signalen mit einer Matrix von Ursprungsquellen verbindet, wobei die Signale modelliert sind als eine Mischung von Komponenten, die jeweils einer zerebralen Ursprungsquelle oder einer artefaktuellen Ursprungsquelle entsprechen;
- Identifizieren und Auswählen wenigstens einer artefaktuellen Augenursprungsquelle aus den separierten Quellen,
- Rekonstruieren wenigstens eines elektroenzephalographischen Signals, das der wenigstens einen identifizierten artefaktuellen Augenursprungsquelle entspricht, durch Anwenden der Mischungsmatrix auf die Matrix von Ursprungsquellen, bei der alle Ursprungsquellen mit Ausnahme der wenigstens einen ausgewählten artefaktuellen Augenquelle annuliert sind;
- Bestimmen von Informationen bezüglich eines Augenblinzelns ausgehend von dem rekonstruierten elektroenzephalographischen Signal.

**13.** Computerprogrammprodukt, umfassend Codebefehle zur Ausführung der Schritte des Verfahrens nach einem der Ansprüche 1 bis 11, wenn das Programm auf einem Computer ausgeführt wird.

**Claims**

**1.** A method for characterising the ocular activity of a person from at least two electro-encephalographic signals delivered by electrodes positioned on the person's scalp, comprising the steps of:

- separating the electro-encephalographic signals into a plurality of origin sources, said signals being modelled as a mixture of components each corresponding to a brain origin source or to an artefact origin source, said separating comprising determining a mixing matrix linking a matrix of electro-encephalographic signals to a matrix of origin sources;
- identifying and selecting at least one ocular artefact origin source from the separated sources,
- reconstructing at least one electro-encephalographic signal corresponding to said selected at least one ocular artefact origin source, said reconstructing comprising applying the mixing matrix to the matrix of origin sources in which all the origin sources, except for said selected at least one ocular artefact source, become zero;
- determining information related to an ocular blink from said reconstructed electro-encephalographic signal.

**2.** The method according to claim 1, wherein identifying the ocular artefact origin source comprises calculating, for each of the separated origin sources, a probability of belonging to an artefact class and/or a probability of belonging to a non-artefact class.

**3.** The method according to claim 2, comprising determining a vector of characteristics for each separated origin source, calculating a probability of belonging to an artefact class and/or a probability of belonging to a non-artefact class being achieved by applying a probability density, set beforehand, to said vector of characteristics.

4. The method according to one of claims 2 and 3, comprising identifying a separated origin source as being an artefact origin source as a function of its probability of belonging to the artefact class or as a function of its probability of belonging to the non-artefact class.

5. The method according to one of claims 1 to 4, wherein identifying the ocular artefact source comprises calculating a time characteristic and/or a spatial characteristic and/or a frequency characteristic and/or an amplitude characteristic of each of the separated origin sources.

6. The method according to claim 5, comprising calculating a time characteristic of the flattening coefficient type or of the dissymmetry coefficient type and/or a spatial characteristic corresponding to an impact and a homogeneity degree of the impact of the source to the person's scalp and/or a frequency characteristic corresponding to a proportion of low frequency components and/or an amplitude characteristic corresponding to a ratio between the samples of a first and a second percentile.

7. The method according to one of claims 1 to 6, wherein at least two electro-encephalographic signals are reconstructed, and wherein for determining information related to an ocular blink, the reconstructed signal having the higher maximum is retained.

8. The method according to one of claims 1 to 7, comprising adaptively amplitude thresholding the reconstructed electro-encephalographic signal retained for determining information related to an ocular blink.

9. The method according to one of claims 1 to 8, wherein determining information related to an ocular blink comprises calculating the derivative of the reconstructed electro-encephalographic signal and searching for instants when said derivative becomes zero.

10. The method according to one of claims 1 to 9, wherein determining information related to an ocular blink comprises determining a blink start, an eyelid closing end and a blink end, and wherein the information related to an ocular blink comprises the blink duration, the maximum blink amplitude, the eyelid closing time, the eyelid opening time.

11. The method according to claim 10, wherein the information related to an ocular blink further comprises the duration between the start and end of a given fraction of the maximum blink amplitude and/or an eyelid opening or closing velocity.

12. A system for characterising the ocular activity of a person from at least two electro-encephalographic signals delivered by electrodes positioned on the person's scalp, comprising processing means configured to:

- separate at least two electro-encephalographic signals of the person into a plurality of origin sources by determining a mixing matrix linking a matrix of electro-encephalographic signals to a matrix of origin sources, said signals being modelled as a mixture of components each corresponding to a brain origin source or to an artefact origin source;
- identifying and selecting at least one ocular artefact origin source from the separated sources,
- reconstructing at least one electro-encephalographic signal corresponding to said identified at least one ocular artefact origin source by applying the mixing matrix to the matrix of origin sources in which all the origin sources, except for said selected at least one ocular artefact source, become zero;
- determining information related to an ocular blink from said reconstructed electro-encephalographic signal.

13. Computer program product comprising code instructions for executing the steps of the method according to one of claims 1 to 11, when said program is executed on a computer.

FIG. 1

FIG. 2

FIG. 3

FIG. 4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **L. SHOKER et al.** Artifact removal from electroencephalograms using a Hybrid BSS-SVM algorithm. *IEEE Signal Process. Lett.,* 2005, vol. 12 (10), 721-724
- **G. BORGHINI et al.** Assessment of mental fatigue during car driving by using high resolution EEG activity and neurophysiologic indices. *Conf. Proc. IEEE Eng. Med. Biol. Soc.,* 2012, vol. 2012, 6442-5
- **ZHIYU QIAN et al.** Analysis of Fatigue with 3D TV based on EEG. *2013 International Conference on Orange Technologies (ICOT),* 12 Mars 2013, 306-309
- **R. ROMO VÁZQUEZ et al.** Blind source separation, wavelet denoising and discriminant analysis for EEG artefacts and noise cancelling. Biomed. *Signal Process. Control,* 2011